# EUROPÄISCHE PATENTSCHRIFT

(11) **EP 1 242 436 B1**
(45) Veröffentlichungstag und Bekanntmachung des Hinweises auf die Patenterteilung: **17.11.2004**
(21) Anmeldenummer: 00983244.5
(22) Anmeldetag: 07.12.2000
(51) Int. Cl.: C07H 3/06

(54) **OLIGOSACCHARIDMISCHUNG**
OLIGOSACCHARIDE MIXTURE
MELANGE D'OLIGOSACCHARIDES

(30) Priorität: 07.12.1999 DE 19958985
(43) Veröffentlichungstag der Anmeldung: 25.09.2002
(73) Patentinhaber: N.V. Nutricia, 2712 HM Zoetermeer (NL)
(72) Erfinder: STAHL, Bernd, 61381 Friedrichsdorf (DE); BÖHM, Günther, 61209 Echzell (DE); FINKE, Berndt, 61239 Ober-Mörlen (DE); GEORGI, Gilda, 61381 Friedrichsdorf (DE); JELINEK, Jürgen, 61191 Rosbach (DE); SCHMITT, Joachim, J., 63768 Hösbach (DE)
(74) Vertreter: Köster, Hajo, Dr.
(86) Internationale Anmeldenummer: PCT/EP2000/012345
(87) Internationale Veröffentlichungsnummer: WO 2001/042263

(56) Entgegenhaltungen:
- EP-A- 0 307 523
- WO-A-98/43494
- DE-A- 19 701 382

## Beschreibung

Die Erfindung betrifft eine Oligosaccharidmischung aus Oligosacchariden, die aus einer oder mehreren Tiermilchen gewonnen wurden und die aus zwei oder mehr Monosaccharideinheiten zusammengesetzt sind, und die Verwendung dieser Oligosaccharidmischung zur Bekämpfung von Störungen gastrointestinaler Funktionen.

Die Konzentration von Oligosacchariden in reifer Humanmilch beträgt etwa 10 g/l. In der Humanmilch konnten bisher etwa 80 verschiedene Oligosaccharide in ihrer Struktur beschrieben werden, die aus 3 - 13 Monomeren zusammengesetzt sind; Newburg, D. S. und Neubauer, S. H. (1995) Carbohydrates in milk: Analysis, quantities and significance, in: Handbook of Milk Composition, Jensen, R. G., editor. Academic press, 273-349. In der Strukturvielfalt dieser Oligosaccharide spielt die Struktur- und Positionsisomerie eine große Rolle. Das Verhältnis von neutralen zu sialisierten (sauren) Oligosacchariden bzw. Oligosaccharidstrukturen beträgt in reifer Humanmilch etwa 10 : 1.

Oligosaccharide spielen beim Menschen bei der spezifischen und unspezifischen Abwehr von Infektionen folgende wichtige Rollen:
1. Oligosaccharide aus Humanmilch stellen eine Substratquelle für Bifidobakterien dar. Damit unterstützen sie die normale, für die Funktion des Gastrointestinaltraktes erforderliche Darmflora und drängen pathogene Keime zurück; Rose, C. S., Kuhn, R., Zilliken, F. und György, P. (1954) Bifidus factor. V. the activity of α- and β-methyl-N-acetyl-D-glueosaminides, *Arch. Biochem. Biophys.* 49, 123-129.
2. Oligosaccharide verhindern die Adhäsion pathogener Keime und/oder Substanzen wie Bakterien, Toxine und eukaryontische Parasiten, indem sie als spezifische Rezeptoranaloga fungieren, wodurch der erste Schritt einer Infektion verhindert wird. In verschiedenen In-vitro-Assays konnte eine anti-adhäsive Wirkung von Oligosacchariden gegenüber verschiedensten pathogenen Mikroorganismen gezeigt werden; Kunz, C. und Rudloff, S. (1993) Biological functions of oligosaccharides in human milk, *Acta paediatr*. 82,, 903-912.
3. Oligosaccharide fördern durch Beeinflussung verschiedener immunologisch wirksamer Zellen die Abwehrkraft gegenüber Infektionen. Die immunmodulatorische Wirkung bestimmter Oligosaccharid-Strukturen konnte am Tiermodell durch eine Erhöhung der Interleukin 10-Produktion gezeigt werden; Velupillai, P. und Harn, D. A. (1994) Oligosaccharidespecific instruction of interleukin 10 production by B 220+ cells from schistosome-infected mice: a mechanism for regulation of CD4+ T-cell subsets, Proc. Natl. Acad. Sci. 91, 1-2.

Die Heterogenität der Oligosaccharide aus den meisten bisher bekannten Säugetiermilchen weicht in vielerlei Hinsicht von humanen Milcholigosacchariden ab. Sowohl quantitativ als auch qualitativ bestehen zahlreiche Unterschiede hinsichtlich der Oligosaccharid-Zusammensetzung. Die Spezies-Unterschiede im Oligosaccharidgehalt von Säugermilchen beziehen sich vornehmlich auf Konzentrationsunterschiede sowohl einzelner Komponenten als auch auf Strukturen mit ähnlichen Merkmalen sowie auch auf die Grundstruktur selbst. Sowohl die Monosaccharidsequenz als auch die Verknüpfung differieren im Vergleich mit den Humanmilch-Oligosacchariden. Zudem ist die Menge der meisten Einzelkomponenten in den meisten tierischen Milchen deutlich geringer als in der menschlichen Muttermilch.

Es wird angenommen, daß diese Unterschiede durch die Anforderungen des Gastrointestinaltraktes des Neugeborenen an die Oligosaccharid-Zusammensetzung der Milch bedingt ist. Diese Anforderungen variieren speziesbedingt sehr stark. Derartige Unterschiede hinsichtlich der Anforderungen gibt es nun nicht nur im Vergleich von einem neugeborenen Kind zu einem neugeborenen Tier sondern auch zwischen verschiedenen neugeborenen Tieren. Die Unterschiede liegen in dem Funktionszustand des Gastrointestinaltraktes der Neugeborenen, in dem Reifungsgrad des Immunsystems, in der speziesabhängigen bevorzugten Nahrung sowie in dem sehr spezifischen Keimmilieu der entsprechenden Spezies begründet.

Versucht man nun Humanmilch durch eine künstliche Milch oder eine infant formula auf Basis von Tiermilchen zu substituieren, dann ist man mit dem Problem konfrontiert, daß die Milch einer einzigen Tierspezies nach heutiger Kenntnislage nicht die heterogene Strukturvielfalt bezüglich der Oligosaccharide besitzt wie diese bei humaner Milch anzutreffen ist.

Aufgabe der vorliegenden Erfindung ist es, eine Oligosaccharidmischung aus Oligosacchariden, die aus einer oder mehreren Tiermilchen gewonnen wurden, bereitzustellen, deren positiven Wirkung und insbesondere deren anti-infektive Wirkung derjenigen von Oligosacchariden aus Humanmilch soweit wie möglich entspricht.

Gelöst wird diese Aufgabe durch eine Oligosaccharidmischung gemäß der Lehre der Ansprüche.

Es wurde überraschend gefunden, daß durch eine neue Mischung von Oligosacchariden aus einer, aber auch aus verschiedenen tierischen Milchen die anti-infektive und/oder präbiotische Wirkung der Tiermilch-Oligosaccharide gesteigert wird. Daher ist die erfindungsgemäße Oligosaccharidmischung aus mindestens zwei Oligosaccharid-Fraktionen aufgebaut, die jeweils mindestens zwei unterschiedliche Oligosaccharide enthalten. Die Oligosaccharid-Fraktionen können dabei aus einer Tiermilch oder auch aus mehreren Tiermilchen stammen. Obwohl es an sich selbstverständlich sein sollte, wird darauf hingewiesen, daß unter dem Tiermilch keine Humanmilch verstanden wird.

Jede der Oligosaccharid-Fraktionen enthält mindestens zwei unterschiedliche Oligosaccharide. Als unterschiedliche Oligosaccharide werden solche angesehen, die sich hinsichtlich mindestens eines Merkmales unterscheiden. So werden beispielsweise zwei Disaccharide, die aus den gleichen Monosaccharideinheiten zusammengesetzt sind, jedoch unterschiedlich verknüpft sind, als unterschiedliche Oligosaccharide angesehen. Die Unterschiede können somit sowohl struktureller Art sein als auch in der Zusammensetzung liegen.

Einer der wesentlichen Aspekte der Erfindung liegt somit darin, Oligosaccharid-Fraktionen aus tierischer Milch einer Spezies bzw. verschiedener Spezies neu zu komponieren, so daß ein erhöhter biologischer Effekt erzielt wird. Natürlich muß bei diesem neuen Komponieren bzw. neuen Mischen sichergestellt werden, daß keine Mischung erhalten wird, die der in einer Tiermilch oder in mehreren Tiermilchen vorhandenen, ursprünglichen Mischung entspricht. Aus diesem Grunde muß sich das Gesamtspektrum der in der Oligosaccharidmischung vorhandenen Oligosacchariden von demjenigen der Tiermilch oder der Tiermilchen aus der oder aus denen die Oligosaccharid-Fraktionen gewonnen wurden, unterscheiden. Wird z. B. die erfindungsgemäße Oligosaccharidmischung nur aus einer Tiermilch gewonnen, dann soll durch letzteres, das Gesamtspektrum betreffende Merkmal zum Ausdruck gebracht werden, daß die neue Komponierung bzw. Mischung der aus dieser einen einzigen Tiermilch gewonnenen Oligosaccharid-Fraktionen nicht wieder zur Ursprungstiermilch führt. Wird die Oligosaccharidmischung aus mehreren Tiermilchen gewonnen, dann darf das Neukomponieren oder Neumischen nicht dazu führen, daß die erhaltene Oligosaccharidmischung einer der Olilgosaccharid-Zusammensetzungen der ursprünglich eingesetzten Tiermilchen entspricht.

In den erfindungsgemäßen Oligosaccharidmischung beträgt das Verhältnis von neutralen Oligosacchariden zu sauren Oligosacchariden 90 - 60 : 10 - 40 Gew.-%. Mit anderen Worten, es liegt eine neutrale Oligosaccharid-Fraktion neben einer sauren Oligosaccharid-Fraktion vor. Beide dieser Fraktionen enthalten mindestens zwei unterschiedliche Oligosaccharide. Durch die Veränderung der Verhältnisse der sauren Oligosaccharid-Fraktion zu der neutralen Oligosaccharid-Fraktion gegenüber dem in der Tiermilch ursprünglich vorhandenen Verhältnisse von neutralen Oligosacchariden zu sauren Oligosacchariden läßt sich eine deutliche Effektivitässteigerung in ihrer physiologischen Wirkung erzielen.

Sofern die Oligosaccharide aus verschiedenen und somit aus mindestens zwei Tiermilchen gewonnen werden, ist eine Neukomponierung bzw. Neumischung derart durchzuführen, dass die aus zwei unterschiedlichen Tiermilchen gewonnenen Oligosaccharide jeweils mindestens 10 Gew.-% der insgesamt der Oligosaccharidmischung vorhandenen Oligosaccharide ausmachen. Werden somit die Oligosaccharide aus zwei Tiermilchen gewonnen, dann müssen die aus einer Tiermilch gewonnenen Oligosaccharide mindestens 10 Gew.-% ausmachen, während die aus der anderen Tiermilch gewonnenen Oligosaccharide ebenfalls mindestens 10 Gew.-% und maximal 90 Gew.-% ausmachen und vice versa. Werden Oligosaccharide hingegen aus drei oder mehr Tiermilchen gewonnen, dann müssen Oligosaccharide von nur zwei Tiermilchen mindestens 10 Gew.-% ausmachen. Die aus den weiteren Tiermilchen gewonnenen Oligosaccharide können weniger als 10 Gew.-% ausmachen.

Als Quelle für die Herstellung der erfindungsgemäßen Oligosaccharidmischung können alle tierischen Milchen eingesetzt werden, wobei Fraktionen aus Kuh-, Ziegen-, Schaf-, Stuten-, Kamel- und Büffelmilch bevorzugt eingesetzt werden.

Die Isolierung der Oligosaccharid-Fraktionen erfolgt mit bekannten Trennverfahren. So kann eine Entfettung mittels Dichtezentrifugation unter Einsatz eines Separators durchgeführt werden. Eine Abtrennung der Proteine kann durch Ultrafiltration bzw. Fällung durch Lösungsmittel (z. B. Ethanol, Aceton) erfolgen. Die Lactose kann durch eine oder mehrere der folgenden Verfahren abgereichert werden: Kristallisation, Filtrationsverfahren sowie chromatographische Verfahren (Ligandenaustausch-Chromatographie, Gelfiltration, Elutionschromatographie, Recycling-Chromatographie und simulierte Gegenstrom-Chromatographie (SMB)). Mineralstoffe werden durch Elektrodialyse, Nanofiltration, Ionenaustausch und/oder Reverse Osmose abgetrennt. Die Fraktionierung der Oligosaccharide in neutrale und saure Oligosaccharide erfolgt mit einer der oben erwähnten chromatographischen Verfahren und/oder der Anionen-Austausch Chromatographie sowie alternativ durch Filtrationsverfahren.

Die erfindungsgemäße Oligosaccharidmischung, deren Oligosaccharide aus mindestens zwei Tiermilchen gewonnen werden, wird derart neu komponiert, daß auch in diesem Falle das Verhältnis von neutralen Oligosacchariden zu sauren Oligosacchariden 90 - 60 : 10 - 40 Gew.-% beträgt.

Mit der Verhältnisangabe in den vorliegenden Unterlagen von 90 - 60 : 10 - 40 Gew.-% sind im übrigen zumindest alle ganzzahligen Zwischenwerte mit umfaßt und offenbart, beispielsweise 89:11, 88:12, 87:13,....85:15,....80:20, 79:21,....75:25, 74:26, 73:27, 72:28,...68:32, 67:33, 66:34, 65:35, 64:36, 63:37, 62:38, 61:39.

Vorzugsweise sind die Oligosaccharide der erfindungsgemäßen Oligosaccharidmischung aus einem oder mehreren der folgenden Monosaccharideinheiten aufgebaut: D-Glucose, D-Galactose, D-N-Acetylglucosamin, D-N-Acetylgalactosamin, L-Fucose, D-Mannose, Sialinsäuren in D-Form (beispielsweise D-N-Acetylneuraminsäure und D-N-Glycolylneuraminsäure) sowie deren O-acetylierte, sulfatierte und phosphorylierte Derivate.

Die in Tiermilchen in großer Menge vorhandene freie Lactose wird im übrigen nicht zu den aus Tiermilchen gewonnenen Oligosacchariden gezählt, welche die erfindungsgemäß eingesetzten Oligosaccharid-Fraktionen ausmachen. Allerdings kann auch in der erfindungsgemäßen Oligosaccharidmischung freie Lactose neben den erfindungsgemäß eingesetzten Oligosaccharid-Fraktionen vorhanden sein.

Mit anderen Worten die erfindungsgemäße Oligosaccharidmischung kann ausschließlich aus den Oligosaccharid-Fraktionen aufgebaut sein, welche Oligosaccharide enthalten, die aus einer oder mehreren Tiermilchen gewonnen wurden und die aus zwei oder mehreren Monosaccharideinheiten zusammengesetzt sind, wobei freie Lactose nicht dazu gehört. Allerdings kann die Oligosaccharidmischung - wie erwähnt-freie Lactose enthalten, stamme sie nun aus Tiermilch oder nicht. Ferner kann die Oligosaccharidmischung ein Oligosaccharid oder mehrere Oligosaccharide enthalten, das bzw. die nicht aus einer Tiermilch stammt bzw. stammen. So kann die erfindungsgemäße Oligosaccharidmischung beispielsweise mit üblichen Sacchariden, die zur Herstellung von Nahrung und insbesondere Babynahrungen eingesetzt werden, vermischt werden. Zudem können in der erfindungsgemäßen Oligosaccharidmischung auch solche Oligosaccharide vorhanden sein, die bei der Gewinnung der erfindungsgemäß eingesetzten Oligosaccharide bzw. Oligosaccharid-Fraktionen durch chemische, enzymatische und/oder technologische Umsetzung aus Glycolipiden und/oder Glycoproteinen freigesetzt wurden.

Wenn im Rahmen der vorliegenden Unterlagen von Oligosacchariden die Rede ist, dann bezieht sich dieser Ausdruck insbesondere auf solche Oligosaccharide, die aus Tiermilchen gewonnen wurden. Daneben können auch andere Oligosaccharide vorhanden sein, wie dies oben dargelegt wurde, wobei dieser Umstand entweder erwähnt ist oder sich aus dem Zusammenhang ergibt.

Eine bevorzugte Quelle zur Oligosaccharid-Isolierung stellen MilchProdukte dar, bei denen sowohl die Protein-Fraktionen als auch das Fett teilweise und/oder vollständig entfernt wurden. Die Aufarbeitung und Fraktionierung kann aus Produkten wie der Lactose-Mineral-Fraktion, die bei der Gewinnung des Rahms, der Kasein-Fraktion und der anderen Inhaltsstoffen anfällt, deutlich vereinfacht werden. Der bei der Herstellung von Pharma-Lactose anfallende Überstand stellt ebenfalls eine geeignete Ausgangsquelle für verschiedene Oligosaccharide dar. Dabei kann, bevor die eigentliche Isolierung der Fraktionen erfolgt, die Milch bzw. Molke von einer Tierspezeis oder von verschiedenen Tierspezies vor der Oligosaccharid-Fraktionierung in dem gewünschten Verhältnis gemischt werden, wie es für die wirksame Oligosaccharidzusammensetzung einzelner Strukturen oder Struktur-Gruppen notwendig ist.

Die erfindungsgemäße Oligosaccharidmischung kann in hochkonzentrierter oder reiner Form bei Störungen gastrointestinaler Funktionen eingesetzt werden, bei denen Dysbakterie (d. h. Störung der gastrointestinalen Flora, z. B. nach Gabe von Antibiotika oder anderen, die Darmflora beeinflussenden Medikamenten nach Nahrungsmittelunverträglichkeit usw.) bzw. virale Infektionen eine Rolle spielen. Sie kann auch zur Prophylaxe solcher gastrointestinaler Infektionen Anwendung finden. Dazu kann die erfindungsgemäße Oligosaccharidmischung einer Babynahrung, einem diätetischen Lebensmittel oder einem Pharmazeutikum einverleibt sein.

Die erfindungsgemäße Oligosaccharidmischung kann auch einen Zusatz zu einer ansonsten kompletten Nahrung darstellen. Derartige Nahrungen sind beispielsweise Säuglingsnahrungen bzw. infant formulae und Präparate zur enteralen klinischen Ernährung. Ferner kann sie als Zusatz zu normalen Nahrungsmitteln zur Erhaltung normaler gastrointestinaler Funktionen (im Sinne einer Prophylaxe) sowie auch zur Verbesserung der gastrointestinalen Funktionen nach Störungen, bei denen bakterielle oder eine virale Fehlbesetzung des Gastrointestinaltraktes eine Rolle spielen, eingesetzt werden. zu 60 % von der Lactose abgereichert. Mittels Gelchromatographie auf Toyopearl HW 40 (S) wird erreicht, daß 5 g einer neutralen Oligosaccharid-Fraktion gewonnen werden, deren Rest-Lactose-Gehalt etwa 10 % beträgt. Die Fraktion der sialysierten Oligosaccharide wird mittels Elektrodialyse vom Anionen-Austausch-Elutionspuffer (Ammoniumacetat) befreit und ebenso gefriergetrocknet wie die neutrale Oligosaccharidfraktion. 4,5 g einer sialysierten Oligosaccharid-Fraktion können auf diese Art gewonnen werden. Durch eine Neukomposition von 1 g der sauren mit 5 g der neutralen Fraktion wird eine aktivere Mischung erzielt als es der natürlichen Zusammensetzung entspricht.

### Beispiel 2

Die bei einer Käseherstellung anfallenden Molken von Ziegen-, Schaf- und Kuhmilch werden, bevor sie aufgearbeitet werden, in einem Verhältnis von 3:1:1 gemischt, so daß eine Gesamtmolke drei verschiedener Tierarten von 4000 I zur Verfügung steht. Durch Ultrafiltration mit einer Polysulphon-Membran (cutoff 20 kDa) wird das Restfett, das in Lösung befindliche Glykomakropeptid und die Molkenproteine abgetrennt. Das Permeat läßt sich in der Folge durch Reverse Osmose auf 900 I konzentrieren. Die weitere Vorgehensweise entspricht der oben beschriebenen, wobei erst die Lactose-Kristallisation und anschließend die Anionen-Austausch-Chromatographie erfolgt mit dem Ziel, Peptide abzutrennen. Die Fraktionen der Elutionsstufen entm. Wasser und 300 mM Ammoniumacetat werden vereinigt, konzentriert, elektrodialysiert und gefriergetrocknet. Es können eine Oligosaccharid-Mischung von drei verschiedenen Spezies in einer Menge von 570 g gewonnen werden. Nach weiterer Trennung mit Gelfiltration und Entsalzung durch Elektrodialyse, kann das Ziel-Produkt durch Gefriertrocknung gewonnen werden. Die Analytik mit Gelfiltration, HPAEC-PAD und MALDI-MS zeigt, daß entsprechend den verwendeten Milchen und des Mischungsverhältnisses das Verhältnis von sauren zu neutralen Oligosacchariden 1:5 beträgt. Der Anteil von Galactosyl-Lactosen kann, bedingt durch den hohen Anteil von Ziegen-Molke, auf 120

Die erfindungsgemäße Oligosaccharidmischung kann als Kapsel bzw. Pulver konditioniert sein oder in verdünnter Form, als Zusatz zu einer ansonsten kompletten Fertignahrung vorliegen.

Die erfindungsgemäße Oligosaccharidmischung wird vorzugsweise in einer Menge von mindestens 10 mg/kg bis 4 g/kg Körpergewicht und Tag eingesetzt, wobei sich die Milligrammwerte auf diejenigen Oligosaccharide in der erfindungsgemäßen Oligosaccharidmischung beziehen, die aus einer Tiermilch gewonnen wurden. Die anderen gegebenenfalls vorhandenen Oligosaccharide wie Lactose und nicht aus einer Tiermilch stammenden Oligosaccharide werden dabei unberücksichtigt gelassen. Insbesondere bevorzugt werden etwa 100 mg/kg und Tag verabreicht.

Die Erfindung wird im folgenden anhand der bevorzugten Ausführungsformen beschreibenden Beispiele näher erläutert.

### Beispiel 1

### 100 I Kuhmilch werden nach folgendem Verfahren aufgearbeitet:

Die Fettabtrennung erfolgt mittels Dichtezentrifugation bei einer Temperatur von 45 °C. Die resultierende Magermilch wird deproteiniert durch eine Ethanol-Fällung (Endkonzentration: 66 % Ethanol). Das ausgefallene Präzipitat wird mit Hilfe eines Separators abgetrennt. Das Ethanol und ein Teil des Wassers werden abgedampf mittels Fallstromverdampfer, so daß ein Volmen von 40 I vorliegt. Darin sind etwa 4 kg Lactose, 1 kg Mineralstoffe und andere lösliche Bestandteile wie wasserlösliche Vitamine, Harnstoff oder Citrat enthalten. Der Anteil der Oligosaccharide beträgt etwa 10 g. Die Zusammensetzung der Oligosaccharidfraktion besteht etwa zu gleichen Teilen aus neutralen und sialysierten Strukturen. Durch Anionen-Austausch Chromatographie auf AG 1 x 2 erfolgt eine Trennung in eine neutrale (Elutionsmittel: entm. Wasser) und eine saure Fraktion (Elutionsmittel: 300 mM Ammoniumacetat). Die neutrale Fraktion wird mittels Kristallisationstechnik g beziffert werden. In der Schafmilch dominiert N-Glycolylneuraminsäure im Verhältnis zu N-Acetylneuraminsäure als wichtiger Bestandteil der sauren Oligosaccharide. Daher wird der Anteil von Glycolylneuraminyl-Oligosacchariden im Vergleich zur Ziegen- und Kuhmilch erhöht.

### Beispiel 3

Es werden parallel die Milchen von Stuten und Ziegen aufgearbeitet. Es stehen jeweils 50 I Milch zur Verfügung. Es wird jeweils eine Kohlenhydrat-Mineralstoff-Fraktion mittels Zentrifugation, ethanolischer Protein-Fällung und Separation gewonnen. Durch den Einsatz eines Rotationsverdampfers werden die Lösungen jeweils aufkonzentriert auf 8 I. Durch Liganden-Austausch Chromatographie auf AG 501 x 8
(Ca²⁺-Form) und Elution mit entm. Wasser gelingt einerseits die chromatographische Abtrennung der Lactose und der Monosaccharide und andererseits eine Subfraktionierung der Oligosaccharide. Aus Stutenmilch wird zwischen dem Elutionsbereich des Ausschlußvolumens und der Tetrasaccharide eine Fraktion gesammelt, die für eine spätere Neukomposition der Oligosaccharide verwendet wird. Die Trennung der Kohlenhydrat-Mineralstoff-Fraktion aus Ziegenmilch auf der gleichen Phase resultiert in eine Lactose- und Monosaccharid abgereicherte Oligosaccharid-Gesamtfraktion. Nachdem eine Entsalzung und gleichzeitige Aufkonzentrierung durch Nanofiltration der jeweiligen Fraktion erfolgt, wird gefriergetrocknet. Beide Fraktionen werden in einem Mischungsverhältnis von 2:5 (Stuten-Oligosaccharid-Fraktion:Ziegen-Oligosaccharid-Fraktion) gemischt.

### Beispiel 4

Ziegenmilch wird wie im Beispiel 1 beschrieben aufgearbeitet. Mittels Anionen-Austausch-Chromatographie auf AG 1 x 2 wird eine saure Fraktion gewonnen, deren Hauptbestandteile 2,3'- und 2,6'-Sialyl-Lactosen sowie 2,3'- und 2,6'-N-Glycolylneuraminyl-Lactose sind. Diese Fraktion wird durch eine Chromatographie auf Reversed Phase-Material weiter aufgetrennt, so daß eine Fraktion bestehend aus 2,3'- und 2,6'-Glycolylneuraminyl-Lactose resultiert. Diese wird durch Elektrodialyse entsalzt, sprühgetrocknet und steht für spätere Mischungen zur Verfügung.

Aus Kuh-Molke gelingt mittels Gelfiltration die Isolierung einer Fraktion von sechs neutralen Trisacchariden: drei isomere Galactosyl-Lactosen, ein fucosyliertes Lactosamin und zwei mit N-Acetylgalactosamin bzw. N-Acetylglucosamin verlängerte Lactose-Einheiten.

Die saure Ziegenmilch-Fraktion, deren dominierende Struktur das 2,3-Isomer bildet, wird im Verhältnis 1:7 mit der neutralen Fraktion der Trisaccharide aus Kuhmolke im trockenen Zustand gemischt und steht für Fütterungsversuche zur Verfügung.

### Beispiel 5

Aus Stutenmilch wird mit den erwähnten Verfahren die Strukturen Lacto-N-neoTetraose sowie Lacto-N-neo-Hexaose isoliert. Diese Oligosaccharide kommen auch in humaner Milch vor.

Aus Kuhmilch wird mit den erwähnten Methoden Disialyl-Lactose chromatographisch isoliert und gefriergetrocknet.

Zwei Positionsisomere eines fucosylierten Tetrasaccharids aus Ziegenmilch werden mit den bekannten etablierten Verfahren isoliert.

In einem Mischungsverhältnis von 2:1:2 (Stutenmilch-OS; Kuhmilch-OS; Ziegenmilch-OS) werden die Oligosaccharide gemischt.

### Beispiel 6

Aus Ziegenmolke kann eine Fraktion neutraler Oligosaccharide isoliert werden, deren Zusammensetzung aus den bereits beschriebenen Tetraosen und desweiteren Penta- bzw. Hexaosen mit bisher unbekannter Monosaccharidzusammensetzung (2 Hexosamine und 4 Hexosen bzw. 2 Hexosamine und 4 Hexosen sowie 3 Hexosamine und 3 Hexosen) besteht.

Aus derselben Aufarbeitung geht eine saure Fraktion hervor, die mit Hilfe der Anionen-Austausch-Chromatographie derart fraktioniert wird, daß eine Mischung mit einer Zusammensetzung der zwei verschiedenen Sialyl-Lactosen und der zwei verschiedenen Glycolylneuraminyl-Lactosen resultiert. Sowohl die längerkettigen sauren Oligosaccharide als auch die neutralen Galactosyl-Lactosen werden zur Neukomposition einer neuen Oligosaccharid-Mischung nicht eingesetzt. Die isolierte saure und die neutrale Fraktion werden im Verhältnis 2:1 gemischt.

### Beispiel 7

Die bei der Käseherstellung anfallende Molke enthält gewöhnlich neben den freien Oligosacchariden auch zahlreiche gebundene Oligosaccharide in Form von N- und O-Glykanen. So ist das Glykomakropeptid O-glycosyliert und das Lactoferrin N-glycosidiert. Die entsprechenden Glykanstrukturen sind im erheblichen Maße sialisiert. Das originäre Verhältnis von sauren zu neutralen Glykanen bzw. Oligosacchariden ist etwa mit 1:1 zu beziffern. Um das Verhältnis zugunsten zu neutralen Oligosacchariden zu verschieben, wird eine Anionen-Austausch-Chromatographie durchgeführt. Alle sialisierten und gebundenen sauren Oligosaccharide (Fraktion 2) binden auf der Gelmatrix und die neutralen Oligosaccharide (Fraktion 1) werden mit entmineralisiertem Wasser eluiert. Alle geladenen Komponenten werden mit hoher Ionenstärke (z.,B. 1 M NaCl) eluiert und weiter bearbeitet. Durch schwache Hydrolysebedingungen bei pH 2,0 und 45 °C mehrere Stunden wird eine Abspaltung der Sialinsäure erreicht. Nach Neutralisation mit NaOH-Lösung wird diese Fraktion 2 mit den neutralen Oligosacchariden (Fraktion 1) vereinigt. Das Verhältnis von neutralen zu sauren Oligosacchariden wird zugunsten der Neutralen verschoben.

## Patentansprüche

1. Oligosaccharidmischung auf Basis von Oligosacchariden, die aus einer oder mehreren Tiermilch(en) gewonnen wurden und die aus zwei oder mehr Monosaccharideinheiten zusammengesetzt sind,
**dadurch gekennzeichnet,**
**dass** sie mindestens zwei Oligosaccharidfraktionen aufweist, die aus jeweils mindestens zwei unterschiedlichen Oligosacchariden aufgebaut sind, wobei freie Lactose nicht dazu gehört,
**dass** sich das Gesamtspektrum der in der Oligosaccharidmischung vorhandenen Oligosaccharide von demjenigen der Tiermilch oder der Tiermilchen, aus der oder aus denen die Oligosaccharidfraktionen gewonnen wurden, unterscheidet,
**dass** das Verhältnis von neutralen Oligosacchariden zu sauren Oligosacchariden 90 - 60 : 10 - 40 Gew.-% beträgt, und
**dass**, sofern die Oligosaccharide aus mindestens zwei Tiermilchen gewonnen wurden, die aus zwei unterschiedlichen Tiermilchen gewonnenen Oligosaccharide jeweils mindestens 10 Gew.-% der insgesamt in der Oligosaccharidmischung vorhandenen Oligosaccharide ausmachen.

2. Oligosaccharidmischung nach Anspruch 1,
**dadurch gekennzeichnet,**
**dass** die Oligosaccharide aus einem oder mehreren der folgenden Monosaccharideinheiten aufgebaut sind: D-Glucose, D-Galactose, D-N-Acetylglucosamin, D-N-Acetylgalactosamin, L-Fucose, D-Mannose, Sialinsäuren in D-Form (D-N-Acetylneuraminsäure und D-N-Glycolylneuraminsäure) sowie deren O-acetylierte, sulfatierte und phosphorylierte Derivate.

3. Oligosaccharidmischung nach Anspruch 1 oder 2,
**dadurch gekennzeichnet,**
**dass** die Oligosaccharide aus einer oder mehreren der folgenden Tiermilchen gewonnen wurden: Kuh-, Ziegen-, Schaf-, Stuten-, Kamelund Büffelmilch.

4. Oligosaccharidmischung nach einem der vorhergehenden Ansprüche,
**dadurch gekennzeichnet,**
**dass** die Oligosaccharide aus ungepoolter oder gepoolter Milch oder Molke oder ungepoolten oder gepoolten Milchprodukten einer oder mehrerer Tierspezies gewonnen wurden.

5. Oligosaccharidmischung nach einem der vorhergehenden Ansprüche,
**dadurch gekennzeichnet,**
**dass** neben den aus einer Tiermilch gewonnen Oligosacchariden bzw. Oligosaccharidfraktionen auch Lactose, die aus der Tiermilch oder den Tiermilchen, aus denen die Oligosaccharide gewonnen wurden, stammen kann, ein oder mehrere, nicht aus einer Tiermilch stammende Oligasaccharid(e) und/oder die bei der Gewinnung der Oligosaccharide durch chemische, enzymatische und/oder technologische Umsetzung aus Glycolipiden und/oder Glycoproteinen freigesetzte Oligosaccharide vorliegen.

6. Oligosaccharidmischung nach einem der vorhergehenden Ansprüche,
**dadurch gekennzeichnet,**
**dass** sie einer Babynahrung, einem diätetischen Lebensmittel oder einem Pharmazeutikum einverleibt ist oder einen Zusatz zu einer ansonsten kompletten Nahrung darstellt.

7. Oligosaccharidmischung nach einem der Ansprüche 1 bis 5 zur Bekämpfung von Störungen gastrointestinaler Funktionen, die durch eine bakterielle oder virale Besiedlung des Gastrointestinaltraktes hervorgerufen wurden, oder zur Erhaltung normaler gastrointestinaler Funktionen.

8. Verwendung einer Oligosaccharidmischung nach einem der Ansprüche 1 bis 5 zur Herstellung eines diätetischen Lebensmittels oder eines Pharmazeutikums zur Bekämpfung von Störungen gastrointestinaler Funktionen, die durch eine bakterielle oder virale Besiedlung des Gastrointestinaltraktes hervorgerufen wurden, oder zur Erhaltung normaler gastrointestinaler Funktionen.

9. Verwendung nach Anspruch 8,
wobei die Oligosaccharidmischung in einer Menge von 10 mg/kg bis 4 g/kg Körpergewicht und Tag eingesetzt wird.

## Claims

1. An oligosaccharide mixture on the basis of oligosaccharides obtained from one or several animal milks and which are composed of two or more monosaccharide units,
**characterized in**
**that** said oligosaccharide mixture contains at least two oligosaccharide fractions built-up by at least two different oligosaccharides with free lactose not pertaining thereto,
**that** the total spectrum of the oligosaccharides present in the oligosaccharide mixture differs from that of the animal milk or animal milks from which the oligosaccharide fractions were obtained,
**that** the ratio of neutral oligosaccharides to acidic oligosaccharides is 90 - 60 : 10 - 40 % by weight, and
**that**, if the oligosaccharides were obtained from at least two animal milks, the oligosaccharides obtained from two different animal milks each amount to at least 10 % by weight of the oligosaccharides present *in toto* in the oligosaccharide mixture.

2. The oligosaccharide mixture according to claim 1,
**characterized in**
**that** the oligosaccharides are built-up by one or several of the following monosaccharide units: D-glucose, D-galactose, D-N-acetyl-glucosamine, D-N-acetyl galactosamine, L-fucose, D-mannose, sialic acids in the D-form (D-N-acetyl neuraminic acid and D-N-glycolyl-neuraminic acid) and the O-acetylated, sulphated and phosphorylated derivatives thereof.

3. The oligosaccharide mixture according to claim 1 or 2,
**characterized in**
**that** the oligosaccharides were obtained from one or several of the following animal milks: cow, goat, sheep, mare, camel and buffalo milk.

4. The oligosaccharide mixture according to any one of the preceding claims,
**characterized in**
**that** the oligosaccharides were obtained from unpooled or pooled milk or lactoserum or from unpooled or pooled milk products of one or several animal species.

5. The oligosaccharide mixture according to any one of the preceding claims,
**characterized in**
**that**, apart from the oligosaccharides or oligosaccharide fractions obtained from an animal milk, lactose is also present, which is allowed to originate from the animal milk or animal milks from which the oligosaccharides were obtained, one or several oligosaccharide(s) not originating from an animal milk are present and/or which are present upon obtainment of the oligosaccharides by chemical, enzymatic and/or technological reaction of oligosaccharides released from glycoproteins and/or glycolipids.

6. The oligosaccharide mixture according to any one of the preceding claims,
**characterized in**
**that** same is incorporated into an infant formula, a dietetic food or a pharmaceutical or constitutes an additive to a nutrition complete *per se.*

7. The oligosaccharide mixture according to any one claims 1 through 5 for combating disorders of gastro-intestinal functions caused by bacterial or viral colonization of the gastro-intestinal tract, or for conserving normal gastro-intestinal functions.

8. The use of the oligosaccharide mixture according to any one claims 1 through 5 for preparing a dietetic food or pharmaceutical composition for combating disorders of gastro-intestinal functions caused by bacterial or viral colonization of the gastro-intestinal tract, or for conserving normal gastro-intestinal functions.

9. The use according to claim 8, whereby the oligosaccharide mixture is being applied in an amount of 10 mg/kg up to 4 g/kg per body weight and day.

## Revendications

1. Mélange d'oligosaccharides à base d'oligosaccharides qui proviennent d'un ou de plusieurs laits animaux et qui sont composés de deux motifs monosaccharides ou plus, **caractérisé en ce qu'**il présente au moins deux fractions d'oligosaccharides qui sont formées à chaque fois d'au moins deux oligosaccharides différents, le lactose libre n'en faisant pas partie,
**en ce que** l'éventail entier des oligosaccharides présents dans le mélange d'oligosaccharides est différent de celui du lait animal ou des laits animaux à partir duquel ou desquels on a obtenu les fractions oligosaccharide,
**en ce que** le rapport des oligosaccharides neutres aux oligosaccharides acides va de 90 à 60:10 à -40% en poids, et
**en ce que** si les oligosaccharides proviennent d'au moins deux laits animaux, les oligosaccharides provenant de deux laits animaux différents représentent à chaque fois au moins 10% en poids de tous les oligosaccharides présents dans le mélange d'oligosaccharides.

2. Mélange d'oligosaccharides selon la revendication 1, **caractérisé en ce que** les oligosaccharides sont formés d'un ou de plusieurs des motifs monosaccharides suivants : le D-glucose, le D-galactose, la D-N-acétylglucosamine, la D-N-acétylgalactosamine, le L-fucose, le D-mannose, les acides sialiques sous forme D (l'acide D-N-acétylneuraminique et l'acide D-N-glycolylneuraminique) ainsi que leurs dérivés O-acétylés, sulfatés et phosphorylés.

3. Mélange d'oligosaccharides selon la revendication 1 ou 2, **caractérisé en ce que** les oligosaccharides proviennent d'un ou de plusieurs des laits animaux suivants : le lait de vache, de chèvre, de brebis, de jument, de chamelle et de bufflonne.

4. Mélange d'oligosaccharides selon l'une quelconque des revendications précédentes, **caractérisé en ce que** les oligosaccharides proviennent de lait ou de lactosérum non groupés ou groupés ou de produits laitiers non groupés ou groupés d'une ou de plusieurs espèces animales.

5. Mélange d'oligosaccharides selon l'une quelconque des revendications précédentes, **caractérisé en ce que**, outre les oligosaccharides ou les fractions d'oligosaccharides provenant d'un lait animal, du lactose, qui peut provenir du lait animal ou des laits animaux à partir duquel ou desquels on a obtenu les oligosaccharides, un ou plusieurs oligosaccharides ne provenant pas d'un lait animal et/ou les oligosaccharides qui ont été libérés de glycolipides et/ou de glycoprotéines lors de la production des oligosaccharides au moyen d'une réaction chimique, enzymatique et/ou technologique, sont présents.

6. Mélange d'oligosaccharides selon l'une quelconque des revendications précédentes, **caractérisé en ce qu'**il est incorporé dans un aliment pour bébés, un aliment diététique ou un produit pharmaceutique, ou qu'il constitue un additif pour un aliment par ailleurs complet.

7. Mélange d'oligosaccharides selon l'une quelconque des revendications 1 à 5 pour lutter contre des troubles des fonctions gastro-intestinales, qui ont été provoqués par une invasion bactérienne ou virale du tractus gastro-intestinal, ou pour entretenir les fonctions gastro-intestinales normales.

8. Utilisation d'un mélange d'oligosaccharides selon l'une quelconque des revendications 1 à 5 pour produire un aliment diététique ou un produit pharmaceutique en vue de lutter contre des troubles des fonctions gastro-intestinales, qui ont été provoqués par une invasion bactérienne ou virale du tractus gastro-intestinal, ou d'entretenir les fonctions gastro-intestinales normales.

9. Utilisation selon la revendication 8, dans laquelle le mélange d'oligosaccharides est mis en oeuvre à raison de 10 mg/kg à 4 g/kg de poids corporel et par jour.
